# EUROPEAN PATENT APPLICATION

(11) **EP 4 656 231 A1**
(43) Date of publication of application: **03.12.2025**
(21) Application number: 24806879.3
(22) Date of filing: 03.04.2024
(51) Int. Cl.: A61M 25/10

(54) **BALLOON CATHETER**

(30) Priority: 18.05.2023 JP 2023082515
(71) Applicant: Goodman Co., Ltd., Aichi 460-0008 (JP)
(72) Inventor: OKAMOTO, Mitsumasa, Seto-shi, Aichi 489-0976 (JP)
(74) Representative: Prüfer & Partner mbB Patentanwälte · Rechtsanwälte
(86) International application number: PCT/JP2024/013694
(87) International publication number: WO 2024/236925

(57) **Abstract**

A balloon catheter includes an inflatable and deflatable balloon 13 and linear protrusions 20 that protrude from a surface of the balloon 13 and extend in an axial direction of the balloon 13 along the surface of the balloon 13. Multiple protrusions 20 are disposed at intervals in a circumferential direction of the balloon 13. The balloon 13 includes wings 22 that are formed when the balloon 13 is deflated. The wings 22 protrude outward in the radial direction of the balloon 13. The wings 22 extend in the axial direction of the balloon 13. Each wing 22 is disposed such that the entire wing 22 is located between the protrusions 20 that are adjacent to each other in the circumferential direction of the balloon 13 in a cross section perpendicular to the axial direction.

## Description

### Cross-Reference to Related Application

The present application is based on Japanese Patent Application No. 2023-082515 filed on May 18, 2023, the entire contents of which are incorporated herein by reference.

### Technical Field

The present disclosure relates to a balloon catheter.

### Background Art

A balloon catheter includes an inflatable and deflatable balloon on a tip side. The balloon catheter is for dilation of a section of a blood vessel that is narrowed or blocked with a lesion by inserting the balloon in a deflated state into the section and then by inflating the balloon.

Another type of balloon catheter includes a linear element on an outer surface of a balloon. The element extends in an axial direction of the balloon catheter. The element protrudes from the outer surface of the balloon. In treatment for the lesion using the balloon catheter with the element, the balloon is inflated and the element is pressed against the lesion. For example, the balloon is inflated such that the element digs into the lesion and creates an incision in the lesion. With the incision used as a trigger of the dilation, the section with the lesion can be easily dilated.

When the balloon of the balloon catheter is deflated, portions of the balloon form wings that protrude outward in a radial direction. Patent literature 1 discloses an example of such a configuration in which wings are formed in a balloon of a balloon catheter with elements when the balloon catheter is deflated. In the balloon catheter in patent literature 1, the wings are disposed to entirely cover the elements.

### Citation List

### Patent Literature

PLT1: International Publication WO2020/012851

### Summary of Invention

### Technical Problem

Because the wings are disposed to entirely cover the elements when the balloon of the balloon catheter in patent literature 1 is deflated, a contour of the balloon is located outer than vertexes of the protrusions by a thickness of the wings. This may reduce insertability of the balloon during insertion of the balloon into a body.

The present disclosure has been made in view of the above circumstances. A main objective of the present disclosure is to provide a balloon catheter that is less likely to cause reduction in insertability of a balloon during insertion of the balloon into a body.

### Solution to Problem

To solve the problem described above, a balloon catheter in a first aspect of the present disclosure includes a balloon and protrusions. The balloon is inflatable and deflatable. The protrusions protrude from a surface of the balloon and extend in an axial direction of the balloon along the surface. The protrusions are disposed at intervals in a circumferential direction of the balloon. The balloon includes a wing that is formed when the balloon is deflated. The wing protrudes outward in a radial direction of the balloon. The wing extends in the axial direction. The wing is disposed such that an entirety of the wing is located between the protrusions that are adjacent to each other in the circumferential direction in a cross section that is perpendicular to the axial direction.

In the first aspect, the balloon includes the wing that is formed when the balloon is deflated. The wing extends in the axial direction of the balloon. In a cross section perpendicular to the axial direction, the wing is disposed such that the entirety of the wing is located between the adjacent protrusions. In such a configuration, the wing is disposed without covering the vertexes of the protrusions. According to the configuration, the outer diameter of the balloon is less likely to increase in contrast to a configuration in which the vertexes of the protrusions are covered with the wing. Therefore, the insertability of the balloon through a body is less likely to decrease.

In a balloon catheter of a second aspect of the present disclosure, the wing in the first aspect includes a bending portion that is bent to protrude in the circumferential direction. The bending portion allows elastic deformation of the wing in the radial direction.

In the second aspect, the wing is elastically deformable in the radial direction of the balloon with the bending portion that is bent to protrude in the circumferential direction. According to the configuration, the wing elastically deforms inward in the radial direction of the balloon when the wing contacts a wall of a tube such as a blood vessel during the insertion of the balloon through the tube. Therefore, the insertability of the balloon is further less likely to decrease.

In a balloon catheter of a third aspect of the present disclosure, the wing in the second aspect includes a portion located outer in the radial direction than vertexes of the protrusions that are adjacent to each other.

In the third aspect, the wing includes the portion located outer in the radial direction than the vertexes of the adjacent protrusions. In such a configuration, the wing is more likely to contact the wall of the tube in the body before the vertexes of the protrusions contact the wall during the insertion of the balloon through the tube. Therefore, the vertexes of the protrusions are less likely to be caught by the wall of the tube. Although the wing has the configuration described above, the wing elastically deforms inward in the radial direction of the balloon when the wing contacts the wall and thus the insertability of the balloon is less likely to decrease.

In a balloon catheter of a fourth aspect of the present disclosure, the wing in any one of the first to the third aspects is bent such that a tip portion of the wing at an end in an extending direction in which the wing extends is covered with a portion of the wing closer to a base portion of the wing than the tip portion in the extending direction. The tip portion is covered from an outer side in the radial direction.

In the fourth aspect, the tip portion of the wing is covered with the portion of the wing closer to the base portion of the wing than the tip portion in the extending direction. The tip portion is covered from the outer side in the radial direction of the balloon. According to the configuration, the tip portion of the wing is less likely to be caught by the wall of the tube during the insertion of the balloon through the tube.

In a balloon catheter of a fifth aspect of the present disclosure, the balloon catheter in any one of the first to the third aspects further includes an inner tube that is inserted through the balloon. The balloon includes a tube-opposed portion that is formed when the balloon is deflated and opposed to an outer periphery of the inner tube. One of the protrusions is defined as a first protrusion and another of the protrusions is defined as a second protrusion. The wing includes a first portion, a second portion, and a third portion. The first portion extends outward from the tube-opposed portion in the radial direction along a side surface of the first protrusion. The second portion extends from an end of the first portion on an outer side in the radial direction toward the second protrusion in the circumferential direction. The third portion extends inward from an end of the second portion on a second protrusion side in the radial direction along a side surface of the second protrusion.

In the fifth aspect, the balloon includes the tube-opposed portion that is formed when the balloon is deflated and opposed to the outer periphery of the inner tube. The wing includes the first portion, the second portion, and the third portion. The first portion extend outward from the tube-opposed portion in the radial direction along the side surface of the first protrusion. The second portion extends from the end of the first portion on the outer side in the radial direction toward the second protrusion in the circumferential direction. The third portion that extends inward from the end of the second portion on the second protrusion side in the radial direction along the side surface of the second protrusion. According to the configuration, dimensions of sections of the vertexes of the adjacent protrusions (the first protrusion and the second protrusion) outer than the wing in the radial direction of the balloon are less likely to be larger. Therefore, the vertexes of the protrusions are less likely to be caught by the wall of the tube during the insertion of the balloon through the tube.

In a balloon catheter of a sixth aspect of the present disclosure, the protrusions in any one of the first to the third aspects are disposed to lean toward the wing when the balloon is deflated.

In the sixth aspect, the dimensions of the sections of the vertexes of the protrusions outer than the wing in the radial direction of the balloon are less likely to be larger because the protrusions are disposed to lean toward the wing when the balloon is deflated. Therefore, the vertexes of the protrusions are less likely to be caught by the wall of the tube during the insertion of the balloon through the tube.

### Brief Description of Drawings

The above-described object and any other object, features, and advantages of the present disclosure will be further clarified by the following detailed description made with reference to the attached drawings.
[FIG. 1] A schematic overall side view illustrating a configuration of a balloon catheter.
[FIG. 2] (a) is a side view illustrating a configuration of a balloon and therearound, and (b) is a cross-sectional view cut along line A-A in (a).
[FIG. 3] A side view illustrating a configuration of the balloon in an inflated state and therearound with longitudinal cross sections of the balloon and an outer tube.
[FIG. 4] A side view illustrating the balloon in a deflated state and therearound.
[FIG. 5] A cross-sectional view cut along line B-B in FIG. 4.
[FIG. 6] Cross-sectional views illustrating other embodiments of wings.
[FIG. 7] A cross-sectional view illustrating a protrusion that leans on a wing.
[FIG. 8] A view illustrating a configuration that includes a notch in a protrusion.

### Description of Embodiments

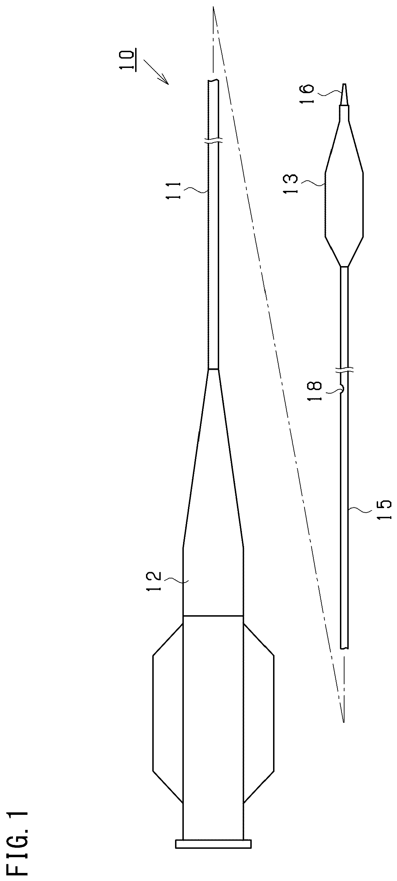

An embodiment of the present disclosure will be described with reference to the drawings. FIG. 1 is a schematic overall side view illustrating a configuration of a balloon catheter.

As illustrated in FIG. 1, a balloon catheter 10 includes a catheter tube 11, a hub 12, and a balloon 13. The hub 12 is mounted to a base portion (a proximal end portion) of the catheter tube 11. The balloon 13 is mounted to a tip portion (a distal end portion) of the catheter tube 11.

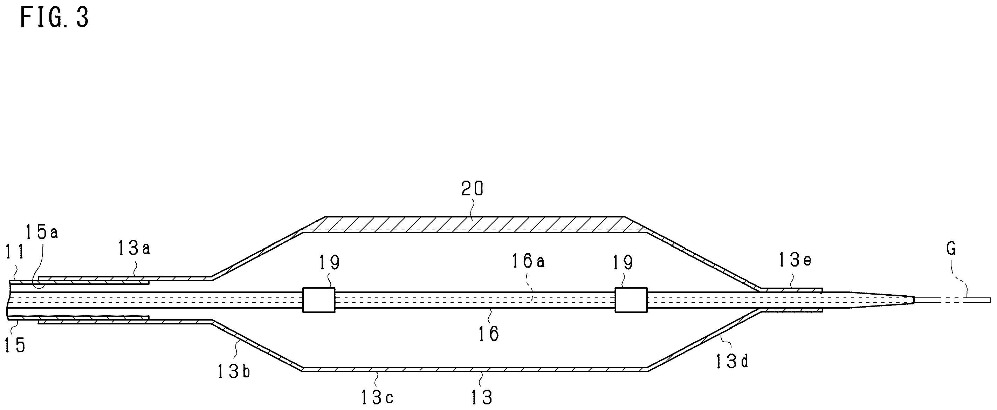

The catheter tube 11 includes an outer tube 15 and an inner tube 16 that is inserted through the outer tube 15. The outer tube 15 is made of a resin material and formed in a tubular shape. The outer tube 15 includes a lumen 15a inside (see FIG. 3). The lumen 15a extends for an entire length of the outer tube 15 in an axial direction of the outer tube 15. A base portion of the outer tube 15 is joined to the hub 12, and a tip portion of the outer tube 15 is joined to the balloon 13. The lumen 15a of the outer tube 15 is communicated with an internal space of the hub 12 and an internal space of the balloon 13. The lumen 15a of the outer tube 15 is a fluid lumen through which a compressed fluid is passed for inflation and deflation of the balloon 13.

The outer tube 15 may be composed of multiple tubes that are disposed in a row in the axial direction and joined to one another. One of the multiple tubes on a base side may be made of a metal material and another of the multiple tubes on a tip side may be made of a resin material.

The inner tube 16 is made of a resin material and formed in a tubular shape. The inner tube 16 includes a lumen 16a inside (see FIG. 3). The lumen 16a extends for an entire length of the inner tube 16 in an axial direction of the inner tube 16. A base portion of the inner tube 16 is joined to a portion of the outer tube 15 at the middle with respect to the axial direction of the outer tube 15. A portion of the inner tube 16 on the tip side extends farther than the tip of the outer tube 15 and inserted in the internal space of the balloon 13. A portion of the inner tube 16 adjacent to the tip portion of the inner tube 16 is joined to a tip portion of the balloon 13.

The lumen 16a of the inner tube 16 is a guidewire lumen through which a guidewire G is inserted. A base opening of the guidewire lumen 16a is located in the middle with respect to the axial direction of the balloon catheter 10. That is, the balloon catheter 10 is classified as an RX-type catheter. A base opening 18 of the guidewire lumen 16a may be located at a base portion of the balloon catheter 10. The balloon catheter 10 may be classified as an over-the-wire-type catheter.

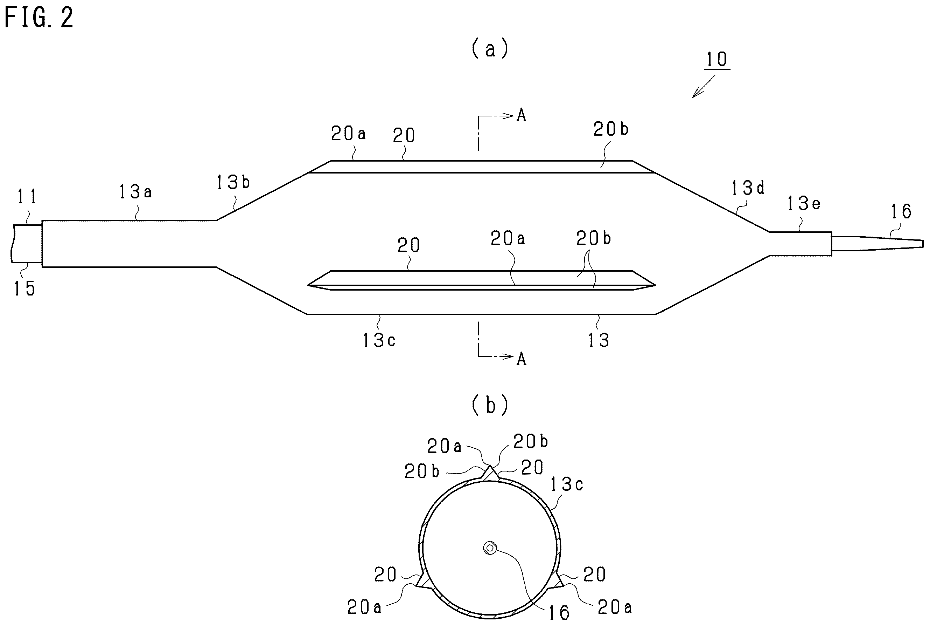

Next, a configuration of the balloon 13 and therearound will be described with reference to FIGS. 2 and 3. FIG. 2(a) is a side view illustrating the configuration of the balloon 13 in an inflated state and therearound. FIG. 2(b) is a cross-sectional view cut along line A-A in FIG. 2(a). FIG. 3 is a side view illustrating the configuration of the balloon 13 in the inflated state and therearound with longitudinal cross sections of the balloon 13 and the outer tube 15.

The balloon 13 is made of a thermoplastic resin material such as polyamide elastomer. As illustrated in FIGS. 2(a) and 3, the balloon 13 is formed in a cylindrical shape (a tubular shape) with a circular cross section as a whole. Specifically, the balloon 13 includes a base leg portion 13a, a base tapered portion 13b, a straight tube portion 13c, a tip tapered portion 13d, and a tip leg portion 13e in this sequence from the base side toward the tip side.

The base leg portion 13a is joined to the tip portion of the outer tube 15. The base tapered portion 13b increases in diameter from the tip of the base leg portion 13a toward the tip side. That is, the base tapered portion 13b is in a tapered shape. The straight tube portion 13c extends from the tip of the base tapered portion 13b toward the tip side with a constant diameter. That is, the straight tube portion 13c is in a circular tube shape (a cylindrical shape). The straight tube portion 13c has a diameter that is to be maximum when the balloon 13 is inflated. The tip tapered portion 13d decreases in diameter from the front end of the straight tube portion toward the tip side. That is, the tip tapered portion 13d is in a tapered shape. The tip leg portion 13e is joined to a portion of the inner tube 16 on the tip side.

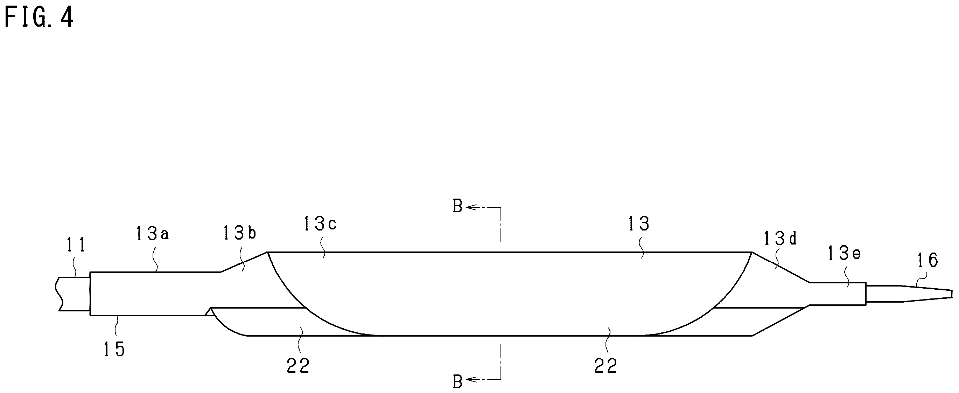

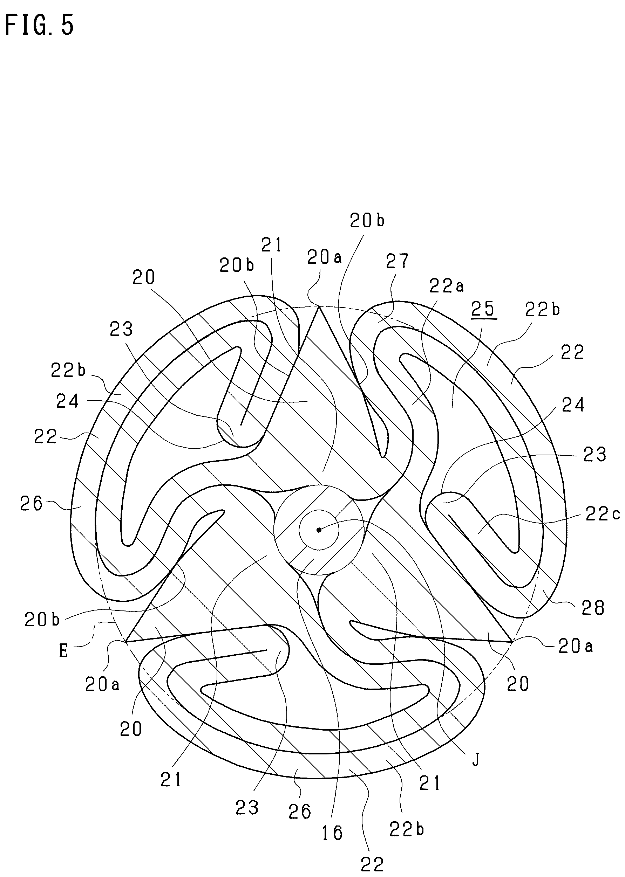

When the compressed fluid is supplied to the internal space of the balloon 13 via the lumen 15a of the outer tube 15, the balloon 13 inflates. When the negative pressure is applied to the lumen 15a of the outer tube 15 and the compressed fluid is discharged from the internal space of the balloon 13, the balloon 13 deflates (see FIGS. 4 and 5).

Two contrast rings 19 are attached to the inner tube 16 inside the balloon 13. The contrast rings 19 are for improving visibility of the balloon 13 in x-ray images to easily position the balloon 13 to a target section for treatment.

The balloon catheter 10 includes linear protrusions 20 on a surface of the balloon 13. The protrusions 20 are for creating incisions in a lesion during dilation of a section with the lesion by inflating the balloon 13. Through the creation of the incisions in the lesion with the protrusions 20, the balloon catheter 10 enables easy dilation of the section with the lesion using the incisions as a trigger of the dilation. That is, the balloon catheter 10 is classified as a balloon catheter having a scoring function.

The protrusions 20 protrude from a surface of the balloon 13. The protrusions 20 extend in the axial direction of the balloon 13 along the surface of the balloon 13. Multiple protrusions 20 are disposed at predetermined intervals (specifically, equal intervals) in the circumferential direction of the balloon 13. This embodiment includes three protrusions 20. Each protrusion 20 is disposed on the straight tube portion 13c to extend for an entire length of the straight tube portion 13c in the axial direction. The protrusions 20 are integrally formed with the balloon 13 and heights of the protrusions 20 from the surface of the balloon 13 are equal.

The protrusions 20 may be disposed on the base tapered portion 13b or the tip tapered portion 13d rather than or in addition to the straight tube portion 13c. In such a configuration in which the protrusions 20 are disposed on the tapered portions 13b, 13d in addition to the straight tube portion 13c, heights of the protrusions 20 on the tapered portions 13b, 13d may be equal to the height of the protrusions 20 on the straight tube portion 13c. Alternatively, the heights of the protrusions 20 on the tapered portions 13b, 13d may be greater than or less than the height of the protrusions 20 on the straight tube portion 13c.

Each protrusion 20 has a transverse section (specifically, a cross section perpendicular to the longitudinal direction of the protrusion 20) in a chevron shape that protrudes outward in the radial direction of the balloon 13, specifically, a triangle shape. Each protrusion 20 includes a vertex 20a at an end of protrusion. The protrusion 20 includes two side surfaces 20b that are adjacent to each other via the vertex 20a.

Next, a configuration of the balloon 13 in the deflated state will be described with reference to FIGS. 4 and 5. FIG. 4 is a side view illustrating the balloon 13 in the deflated state and therearound. FIG. 5 is a cross-sectional view cut along line B-B in FIG. 4.

As illustrated in FIGS. 4 and 5, the balloon 13 includes tube-opposed portions 21 and wings 22 that are formed when the balloon 13 is deflated. The tube-opposed portions 21 are opposed to the outer periphery of the inner tube 16 that is placed inside balloon 13. The tube-opposed portions 21 extend in the circumferential direction of the inner tube 16 (or the circumferential direction of the balloon 13) along the outer periphery of the inner tube 16.

The wings 22 extend outward from the tube-opposed portions 21 in the radial direction of the balloon 13 (hereinafter may be referred to as a balloon radial direction). Multiple wings 22 are disposed at predetermined intervals (specifically, equal intervals) in the circumferential direction of the balloon 13. This embodiment includes three wings 22, that is, the number of the wings 22 and the number of the protrusions 20 are equal. Each wing 22 extends in the axial direction over the tapered portion 13b, 13d and the straight tube portion 13c of the balloon 13. The wings 22 have the same shape and size.

Portions of the balloon 13 are folded and formed into the wings 22. The wings 22 include tip portions 23 that are at ends in extending directions in which the wings 22 extend from the tube-opposed portions 21. The tip portions 23 include creases 24 that extend in the axial direction of the balloon 13. The wings 22 are folded along the creases 24.

Multiple, specifically three, tube-opposed portions 21 are disposed in the circumferential direction of the balloon 13 as with the wings 22. That is, the number of the tube-opposed portions 21 and the number of the wings 22 are equal. The tube-opposed portions 21 are provided for the wings 22, respectively. Each tube-opposed portion 21 extends between the adjacent wings 22 to connect one of the adjacent wings 22 to another.

The balloon catheter 10 has a distinctive feature in the configuration of the wings 22. The distinctive feature will be described.

As illustrated in FIG. 5, each wing 22 is disposed between the adjacent protrusions 20 in the circumferential direction of the balloon 13 (hereinafter may be referred to as a balloon circumferential direction). Specifically, each wing 22 is disposed such that the entire wing 22 is located between the adjacent protrusions 20 in a cross section perpendicular to the axial direction of the balloon 13 (i.e., the transverse section). In such a configuration, each wing 22 does not cover the vertex 20a of the protrusion 20 from an outer side in the balloon radial direction. Because each wing 22 is disposed as described above, the wings 22 and the protrusions 20 alternately appear in the circumferential direction of the balloon 13.

Each wing 22 is disposed such that the wing 22 is sandwiched between the protrusions 20 that are adjacent to each other in the balloon circumferential direction. One of the adjacent protrusions 20 and another of the adjacent protrusions 20 may be referred to as a first protrusion 20 and a second protrusion 20, respectively. Each wing 22 includes a first portion 22a, a second portion 22b, and a third portion 22c. The first portion 22a extends outward from the tube-opposed portion 21 in the balloon radial direction along a side surface 20b of the first protrusion 20 (corresponding to a first protrusion). The second portion 22b extends from an end of the first portion 22a on the outer side in the balloon radial direction toward the second protrusion 20 (corresponding to a second protrusion) in the balloon circumferential direction. The third portion 22c extends inward from an end of the second portion 22b on a second protrusion 20 side in the balloon radial direction along a side surface 20b of the second protrusion 20. The first portion 22a contacts the side surface 20b of the first protrusion 20 and the third portion 22c contacts the side surface 20b of the second protrusion 20. An end of the third portion 22c on an opposite side from the second portion 22b side is defined as a tip portion 23 of the wing 22.

The second portion 22b is located at about the same position as the vertex of each protrusion 20 with respect to the balloon radial direction. When an imaginary circle E that has the axis J of the balloon 13 as a center (a center axis) and passes the vertexes 20a of the protrusions 20 is drawn (see two-dot chain line in FIG. 5) in a cross section perpendicular to the axial direction of the balloon 13 (i.e., a transverse section), the second portion 22b extends in an arc along the imaginary circle E. Specifically, the second portion 22b includes a stray section 26 that is located outer than the imaginary circle E in the balloon radial direction. The stray section 26 of the second portion 22b is located outer than the vertex 20a of the protrusion 20 in the balloon radial direction. A dimension of the stray section 26 (i.e., a length measuring in the balloon radial direction) is less than the thickness of the wing 22. The dimension is about equal to the thickness of the balloon 13 (i.e., a half of the thickness of the wing 22) in this embodiment.

The second portion 22b of the wing 22 includes bending sections 27 and 28 at ends with respect to the balloon circumferential direction. The bending section 27 is at a border between the second portion 22b and the first portion 22a. The bending section 28 is at a border between the second portion 22b and the third portion 22c. The bending sections 27 and 28 are bent to protrude toward opposite sides from each other in the balloon circumferential direction. Specifically, the bending sections 27 and 28 are curved to protrude toward the opposite sides from each other. With the bending sections 27 and 28, the wing 22 is elastically deformable in the balloon radial direction. Because the bending section 28 is provided in the wing 22, the tip portion 23 of the wing 22 is covered with the second portion 22b from the outer side in the balloon radial direction. The second portion 22b corresponds to "a portion of the wing 22 closer to a base portion of the wing 22 than the tip portion 23 in an extending direction in which the wing 22 extends."

Because the bending sections 27 and 28 are provided in the wing 22, the wing 22 forms a circular shape. A predefined space 25 is defined inside the wing 22. The space 25 is surrounded by the first portion 22a, the second portion 22b, and the third portion 22c of the wing 22.

Next, a method of using the balloon catheter 10 will be descried. Specifically, a procedure for dilating a section of a blood vessel with a lesion using the balloon catheter 10 will be described.

First, a guiding catheter is inserted through a sheath introducer in the blood vessel until a tip opening of the guiding catheter reaches a coronary ostium. Then, a guide wire G is inserted through the guiding catheter until the guide wire G reaches a distal point via the coronary ostium and the section with the lesion.

Next, the balloon catheter 10 is inserted through the guiding catheter along the guide wire G. After the insertion, the balloon 13 is advanced to (or disposed in) the section with the lesion while being pushed back and forth. During the insertion, the balloon 13 is maintained in the deflated state. When the balloon 13 is deflated, the wings 22 are formed in the balloon 13 as described above. Each wing 22 is disposed such that the entire wing 22 is located between the adjacent protrusions 20 in the transverse section (see FIG. 5). In this state, each wing 22 is disposed without covering the vertex 20a of the protrusion 20. According to the configuration, the outer diameter of the balloon 13 is less likely to increase in contrast to a configuration in which the vertexes 20a of the protrusions 20 are covered with the wings 22. Therefore, the insertability of the balloon 13 through the blood vessel is less likely to decrease.

When the balloon 13 reaches the section with the lesion, the balloon 13 is inflated. The protrusions 20 are pressed against the lesion and incisions (cracks) are created with the protrusions 20. With the incisions used as a trigger of the dilation, the section with the lesion can be dilated.

When the dilation of the section with the lesion with the balloon 13 is complete, the balloon 13 is deflated. With the balloon 13 in the deflated state, the balloon catheter 10 is pulled out of the body. This completes steps of the procedure.

As described above, the balloon catheter 10 is generally used for blood vessel treatment, for example, treatment for coronary arteries, femoral arteries, pulmonary arteries, or other types of blood vessels; however, the balloon catheter 10 can be used for treatment of any other types of tubes in living organism other than blood vessels such as ureters and gastrointestinal tracts or body cavities.

According to the configuration of the embodiment described above in detail, the following advantageous effects can be achieved.

The wings 22 include the bending sections 27, 28 that are bent to protrude in the circumferential direction of the balloon 13. With the bending sections 27, 28, the wings 22 are elastically deformable in the radial direction of the balloon 13. According to the configuration, the wings 22 (specially the second portions 22b) deform inward in the radial direction of the balloon 13 when the wings 22 contact a wall of the blood vessel and the wall of the blood vessel pushes back the wings 22 during insertion of the balloon 13 through the blood vessel. Therefore, the insertability of the balloon 13 is further less likely to decrease.

The wings 22 include portions located outer than the vertexes 20a of the corresponding protrusions 20 in the balloon radial direction (specifically, the stray sections 26). The corresponding protrusions 20 are adjacent to the wings 22 in the balloon circumferential direction. According to the configuration, the wings 22 are more likely to contact the wall of the blood vessel before the vertexes 20a of the protrusions 20 contact the wall of the blood vessel during the insertion of the balloon 13 through the blood vessel. Therefore, the vertexes 20a of the protrusions 20 are less likely to be caught by the wall of the blood vessel. Although the wings 22 have the configuration described above, the insertability of the balloon 13 is less likely to decrease because the wings 22 elastically deform inward in the radial direction of the balloon 13 when the wings 22 contact the wall of the blood vessel.

The wings 22 include the tip portions 23 that are bent such that the tip portions 23 are covered with portions of the wings 22 located closer to the base portions than the tip portions 23 (specifically, the second portions 22b) from the outer side in the radial direction of the balloon 13. According to the configuration, the tip portions 23 of the wings 22 are less likely to be caught by the wall of the blood vessel during the insertion of the balloon 13 through the blood vessel.

The balloon 13 includes the tube-opposed portions 21 that are formed when the balloon 13 is deflated and are opposed to the outer periphery of the inner tube 16. Here, one of the protrusions 20 that are adjacent to the wing 22 is defined as the first protrusion 20 and the other one is defined as the second protrusion 20. The wing 22 includes the first portion 22a, the second portion 22b, and the third portion 22c. The first portion 22a extends outward from the tube-opposed portion 21 in the balloon radial direction along the side surface 20b of the first protrusion 20. The second portion 22b extends from the end of the first portion 22a on the outer side in the balloon circumferential direction toward the second protrusion 20 in the balloon circumferential direction. The third portions 22c extends inward from the end of the second portion 22b on the second protrusion 20 side in the balloon radial direction along the side surface 20b of the second protrusion 20. According to the configuration, the dimensions of sections of the vertexes of the adjacent protrusions 20 outer than the wing 22 in the balloon radial direction are less likely to be larger. Therefore, the vertexes 20a of the protrusions 20 are less likely to be caught by the wall of the blood vessel during the insertion of the balloon 13 through the blood vessel.

The present disclosure is not limited to the above embodiment and may be implemented as the followings, for example.
(1) In the above embodiment, each wing 22 includes the portion located outer than the vertexes 20a of the adjacent protrusions 20 in the balloon radial direction (specifically, the stray portion 26). The adjacent protrusions 20 are adjacent to each other in the balloon circumferential direction. However, the wing 22 may be configured without the stray portion 26. For example, outer edges of each wing 22 and the vertexes 20a of the adjacent protrusions 20 may be at the same position with respect to the balloon radial direction. Namely, the wings 22 may contact the imaginary circle E.

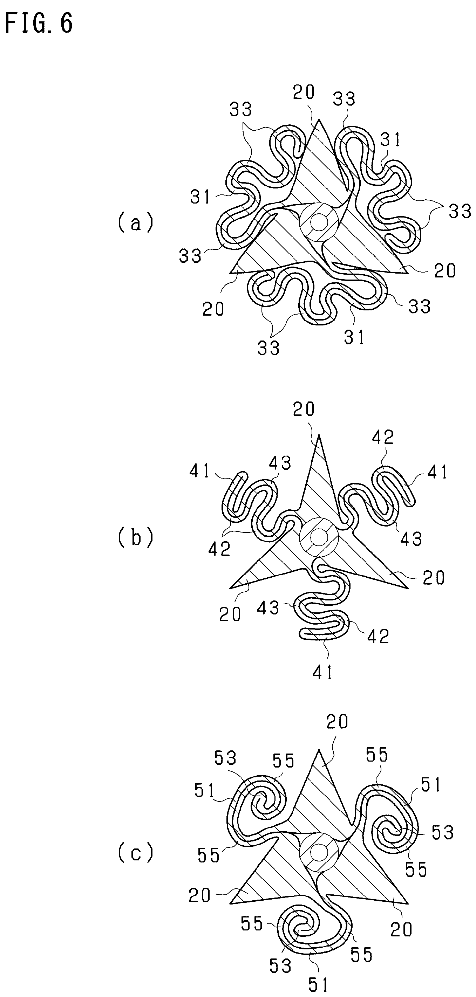

(2) FIGS. 6(a) to 6(c) illustrates other embodiments of wings. Wings 31 illustrated in FIG. 6(a) wavily extend in the circumferential direction of the balloon 13. In this configuration, each wing 31 includes multiple bending portions 33 that protrude outward in the radial direction of the balloon 13. According to the configuration, the bending portions 33 of the wings 31 contact the wall of the blood vessel during the insertion of the balloon 13 through the blood vessel. That is, a contact area of the wings 31 that contact the wall of the blood vessel is reduced. Therefore, easiness in insertion of the balloon 13 through a narrowed section of the blood vessel improves.
(3) Wings 41 illustrated in FIG. 6(b) wavily extend in the radial direction of the balloon 13. In this configuration, the wings 41 include bending portions 42 that are bent to protrude toward one side in the circumferential direction of the balloon 13 and bending portions 43 that are bent to protrude toward the other side in the circumferential direction of the balloon 13. With the bending portions 42, 43, the wings 41 are elastically deformable in the radial direction of the balloon 13. When the wings 41 contact the wall of the blood vessel during the insertion of the balloon 13 through the blood vessel, the wings 41 elastically deform inward in the radial direction of the balloon 13. Therefore, the insertability of the balloon 13 through the blood vessel is further less likely to decrease.
(4) Wings 51 illustrated in FIG. 6(c) are wound such that spiral forms are shown in a cross section perpendicular to the axial direction of the balloon 13 (i.e., a transverse section). In this configuration, a tip portion 53 of each wing 51 is located at the center of the spiral form. According to the configuration, the tip portions 53 of the wings 51 are less likely to be caught by the wall of the blood vessel during the insertion of the balloon 13 through the blood vessel.

Because the wings 51 are wound, the wings 51 include curved portions 55 that are curved to protrude in the circumferential direction of the balloon 13. With the curved portions 55, the wings 51 are elastically deformable in the radial direction of the balloon 13. When the wings 51 contact the wall of the blood vessel, the wings 51 elastically deform inward in the radial direction. According to the configuration, the insertability of the balloon 13 is further less likely to decrease during the insertion of the balloon 13 through the blood vessel.

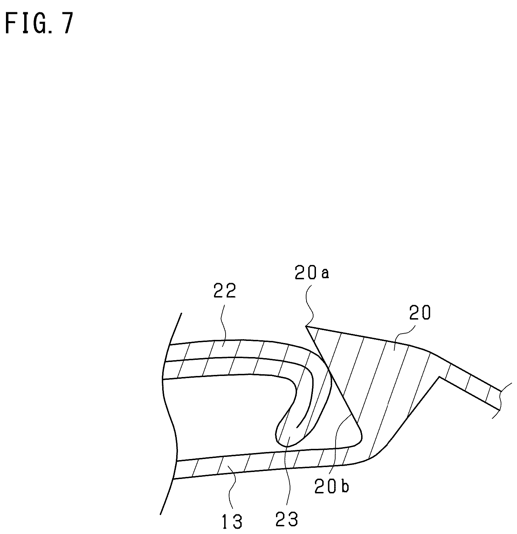

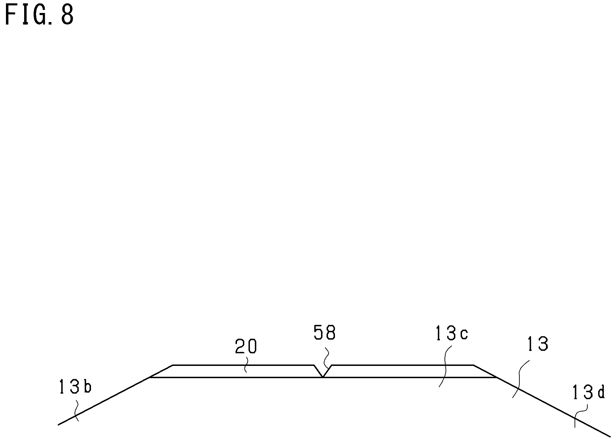

Because the wings 51 are wound, the contact area of the wings 51 that contact the wall of the blood vessel is reduced. This reduces sliding resistance of the wings 51 that slide on the wall of the blood vessel during the insertion of the balloon 13 through the narrowed section of the blood vessel. Therefore, the easiness in insertion of the balloon 13 through the narrowed section improves.
(5) In the above embodiment, the second portion 22b of the wings 22 and the vertexes 20a of the protrusions 20 are at about the same position with respect to the balloon radial direction. However, the second portions 22b of the wings 22 may be located inner in the balloon radial direction than the vertexes 20a of the protrusions 20. In this configuration, the vertexes 20a of the protrusions 20 are located outer in the balloon radial direction than the wings 22. As illustrated in FIG. 7, the protrusions 20 may be disposed to lean toward the wings 22 when the balloon 13 is in the deflated state. In FIG. 7, the protrusion 20 leans on the wing 22. In this configuration, a dimension of a section of the vertex 20a of the protrusion 20 outer in the radial direction of the balloon 13 than the wing 22 is less likely to be larger. Therefore, the vertex 20a of the protrusion 20 is less likely to be caught by the wall of the blood vessel during the insertion of the balloon 13 through the blood vessel.
(6) As illustrated in FIG. 8, the protrusions 20 of the balloon 13 may include notches 58. Each notch 58 is formed in an intermediate portion of the protrusion 20 with respect to the longitudinal direction, specifically, at the middle with respect to the longitudinal direction. In this configuration, the protrusions 20 can easily bend with the notch 58 as a start point of the bending. This allows the balloon 13 to easily follow a curve of a curved blood vessel during the insertion of the balloon 13 through the curved blood vessel.

In a known configuration in which the vertexes 20a of the protrusions 20 are covered with the wings 22 when the balloon 13 is deflated, the bending of the protrusions 20 with the notches 58 as starting points of the bending may be disturbed by the wings 22. This may make the balloon 13 difficult to follow the curve of the curved blood vessel during the insertion of the balloon 13 through the curved blood vessel. According to the configuration in this disclosure in which each wing 22 is disposed between the adjacent protrusions 20, the bending of the protrusion 20 is not disturbed by the wing 22. Therefore, the balloon 13 can easily follow the curve of the curved blood vessel.

(7) In the above embodiment, the protrusions 20 are used to create incisions in the lesion. However, the protrusions 20 may be used for other purposes. For example, the balloon 13 may be inflated in the blood vessel until the protrusions 20 dig into the wall of the blood vessel and use the protrusions 20 for anti-slip purpose to restrict the balloon 13 from slipping.

The present disclosure has been described with reference to the embodiments. However, the present disclosure is not limited to the embodiments or the structures. Various modifications and modifications within a scope of equivalents may be within in the technical scope of the present disclosure. Further, various combinations, configurations, and other combinations and configurations including single elements, more or less, may be within the technical scope of the present disclosure.

### Reference Sings List

10: Balloon catheter, 13: Balloon, 16: Inner tube, 20: Protrusion, 20a: Vertex, 21: Tube-opposed portion, 22: Wing, 27: Bending portion, 28: Bending portion

## Claims

1. A balloon catheter comprising:
a balloon that is inflatable and deflatable; and
protrusions that protrude from a surface of the balloon and extend in an axial direction of the balloon along the surface, wherein
the protrusions are disposed at intervals in a circumferential direction of the balloon,
the balloon includes a wing that is formed when the balloon is deflated,
the wing protrudes outward in a radial direction of the balloon,
the wing extends in the axial direction, and
the wing is disposed such that an entirety of the wing is located between the protrusions that are adjacent to each other in the circumferential direction in a cross section that is perpendicular to the axial direction.

2. The balloon catheter according to claim 1, wherein the wing includes a bending portion that is bent to protrude in the circumferential direction and that allows elastic deformation of the wing in the radial direction.

3. The balloon catheter according to claim 2, wherein the wing includes a portion that is located outer in the radial direction than vertexes of the protrusions that are adjacent to each other.

4. The balloon catheter according to any one of claims 1 to 3, wherein the wing is bent such that a tip portion of the wing at an end in an extending direction in which the wing extends is covered with a portion of the wing close to a base portion of the wing than the tip portion in the extending direction, wherein the tip portion is covered from an outer side in the radial direction.

5. The balloon catheter according to any one of claims 1 to 3, further comprising an inner tube that is inserted through the balloon, wherein
the balloon includes a tube-opposed portion that is formed when the balloon is deflated and opposed to an outer periphery of the inner tube,
one of the protrusions is defined as a first protrusion and another of the protrusions is defined as a second protrusion, and
the wing includes:
a first portion that extends outward from the tube-opposed portion in the radial direction along a side surface of the first protrusion;
a second portion that extends from an end of the first portion on an outer side in the radial direction toward the second protrusion in the circumferential direction; and
a third portion that extends inward from an end of the second portion on a second protrusion side in the radial direction along a side surface of the second protrusion.

6. The balloon catheter according to any one of claims 1 to 3, wherein the protrusions are disposed to lean toward the wing when the balloon is deflated.
